Europäisches Patentamt

European Patent Office

Office européen des brevets

(11) Veröffentlichungsnummer: **0 349 712**

**A1**

(12) # EUROPÄISCHE PATENTANMELDUNG

(21) Anmeldenummer: 89105573.3

(22) Anmeldetag: 30.03.89

(51) Int. Cl.4: **A23L 1/30 , A23C 9/12 , C13K 13/00 , A23L 2/26**

Die Bezeichnung der Erfindung wurde geändert (Richtlinien für die Prüfung im EPA, A-III, 7.3).

(30) Priorität: 11.04.88 DE 3811964
06.05.88 DE 3815513

(43) Veröffentlichungstag der Anmeldung:
10.01.90 Patentblatt 90/02

(84) Benannte Vertragsstaaten:
AT BE CH DE ES FR GB GR IT LI LU NL SE

(71) Anmelder: **Biodyn AG**
**Industriestr. 31**
**CH-8305 Dietlikon(CH)**

(72) Erfinder: **Lembke, Andreas, Prof.Dr.Dr.**
**Eutiner Strasse 1**
**D-2420 Eutin-Sielbeck(DE)**
Erfinder: **Strobel, Hans Joachim, Dr.**
**Opfikonerstrasse 33**
**CH-8303 Bassersdorf(CH)**

(74) Vertreter: **Hach, Hans Karl, Dr.**
**Tarunstrasse 23**
**D-6950 Mosbach-Waldstadt(DE)**

(54) **Nahrungsmittel oder Getränk.**

(57) Ein Nahrungsmittel enthält 2 bis 10 TGT (Trockengewichtsteile) Galactose auf 100 TGT übrigen Zucker.

EP 0 349 712 A1

EP 0 349 712 A1

## NAHRUNGSMITTEL ODER GENUSSMITTEL FÜR MENSCHLICHE ERNÄHRUNG

Die Erfindung betrifft ein Nahrungsmittel oder Genußmittel, vorzugsweise Getränk, für menschliche Ernährung mit einem Gehalt an Zucker, mit einem Gehalt an Galactose in HÖhe eines Bruchteils des übrigen Zuckergehaltes zur Vermeidung der zahnschädigenden Wirkungen dieses Zuckers.

Aus der EU 0184121-A2 ist es bekannt, einem saccharosehaltigen Nahrungsmittel Galactose zuzusetzen, um damit die kariesfördernde Wirkung der Saccharose zu behindern. Diese Behinderung beruht auf der Tatsache, daß Galactose Haftrezeptoren für die säurebildenden, Karies hervorrufenden Bakterien auf der Zahnoberfläche belegt, wodurch den Bakterien die Möglichkeit genommen wird, an der Zahnoberfläche Halt zu finden.

Außer Saccharose haben auch andere Zucker, wie Glucose, Fructose und Maltose kariesfördernde Wirkung.

Aufgabe der Erfindung ist es, unter Ausnutzung der genannten Schutzwirkung der Galactose die kariesfördernde Wirkung aller mit der Nahrung zugeführter Zucker zu behindern, und zwar unter möglichst weitgehender Vermeidung unerwünschter Nebenwirkungen besonders im Verdauungssystem.

Die Erfindung ist dadurch gekennzeichnet, daß auf 100 Trockengewichtsteile (TGT) Gehalt an übrigem Zucker (Gesamtzucker, ausgenommen Galactose) 2 bis 10 TGT, vorzugsweise 5 TGT, Galactose als Zusatz eingesetzt werden, und zwar unter Anrechnung der eventuell bereits vorhandenen Galactose.

Der kariöse Angriff findet auf der Zahnoberfläche statt, wo bei die Bakterien in einem Belag des Zahnes, dem sogenannten Plaque, ihren Stoffwechsel vollführen. Ein niedriger pH-Wert im Plaque begünstigt die kariöse Wirkung.

Bei vielen Nahrungsmitteln, insbesondere Getränken, wie Fruchtsaftgetränken, ist ein niedriger pH-Wert aus geschmacklichen Gründen wünschenswert. Deshalb empfiehlt es sich, insbesondere bei Getränken, einer Senkung des pH-Wertes im Plaque durch das zugeführte Nahrungsmittel entgegenzuwirken, indem man das Nahrungsmittel beziehungsweise Getränk puffert. Dabei sollen unerwünschte Nebenwirkungen, insbesondere hinsichtlich des Geschmacks vermieden werden.

Zu diesem Zweck setzt man zweckmäßig als Puffersubstanz Phosphat und/oder Citrat, vorzugsweise in Form von Alkalisalz, ein, da diese Puffersubstanzen in der Nahrung unschädlich, geschmacklich tolerabel und im fraglichen Pufferbereich wirksam sind. Eine unerwünschte Absenkung des pH im Plaque wird mit solchen Puffersubstanzen erzielt, wenn die Pufferkapazität entsprechend eingestellt ist. Zu diesem Zweck wird die Pufferkapazität definiert nach der Gleichung $KaPx = y$, wobei $KaPx$ die Pufferkapazität für $pHx$ und $y$ die Anzahl der ml (Milliliter) an 0,1 nHCL (Normal-Salzsäure) angibt, die nötig sind, um 100 ml einer auf pH 7 eingestellten Lösung auf $pHx$ umzustellen. Nach dieser Definition wird die Pufferkapazität KaP 6,0 eingestellt auf 10 bis 45, vorzugsweise 20 bis 41.

In diesem Zusammenhang hat sich gezeigt, daß durch einen wenig sauren oder neutralen pH-Wert im Plaque auch die Schutzwirkung der Galactose begünstigt wird im Sinne eines synergetischen Effektes.

Die Galactose kann in reiner Form eingesetzt werden. Aus wirtschaftlichen Gründen empfiehlt es sich, die Galactose in Form eines Lactosehydrolysationsproduktes - im folgenden kurz LHP genannte - einzusetzen, das gewonnen ist, indem Lactose mit Beta-Galactosidase hydrolysiert ist und die so behandelte Lactose mit ihrem gesamten Zuckergehalt als LHP eingesetzt ist.

Die Hydrolysation kann unter Einsatz von Mikroorganismen erfolgen. Für diesen Fall empfiehlt es sich, daß die Mikroorganismen nach der Hydrolysation und vor dem Einsatz des LHP entfernt werden.

Werden zur Hydrolysation der Lactose Mikroorganismen angewandt, dann verbrauchen diese bei der Hydrolyse einen großen Teil der durch die Hydrolyse neben der Galactose anfallenden Glucose. Man gewinnt dann ein LHP, dessen Zuckerbestandteile, bezogen auf 100% (Prozent), beispielsweise umfassen: 40 % Lactose, 45 % Galactose und 15 % Glucose. Eine weitergehende Spaltung der Lactose, die im Interesse einer hohen Galactoseausbeute wünschenswert wäre, erfordert bei der Hydrolyse mit Mikroorganismen aber untragbar hohen Aufwand bei der Gestaltung und dem Betrieb des Fermenters, unter anderem bedingt durch die Notwendigkeit, Sterilbedingungen einzuhalten. Aus diesem Grunde wird der Einsatz von Beta-Galactosidase vorzugsweise in Form des Enzyms EC 3.2.1.23 zur Hydrolysation gegenüber dem Einsatz von Mikroorganismen bevorzugt.

Bei enzymatischer Hydrolyse ist mit tragbarem technischen Aufwand ein hoher Hydrolysegrad erzielbar und es ist deshalb bei enzymatischer Hydrolyse bevorzugt, daß in dem eingesetzten LHP 60 bis 85%, vorzugsweise 75 bis 80%, der Lactose hydrolysiert ist.

Der bei enzymatischer Hydrolyse anfallende höhere Glucosean teil ist nicht schädlich im LPH, da die Glucose zur Süßung beiträgt und ihre kariesfördernde Wirkung kompensiert werden kann, indem man diesen Glucoseanteil bei der Bemessung der dem Nahrungsmittel zuzusetzenden Galactosemenge berück-

2

sichtigt und zwar auch dann, wenn diese Galactose als Bestandteil eines LHP zugegeben wird.

Zur Gewinnung des LHP geht man aus wirtschaftlichen Gründen vorzugsweise von Molke aus, entweder als wiederaufgelöstes Molkepulver oder in Form flüssiger Molke, so wie sie beispielsweise bei der Käserei anfällt.

Molkeeiweiß hat sich in dem LHP in vielen Fällen als nachteilig erwiesen, weil es zum Beispiel bei Getränken zu einer unerwünschten Trübung führt. Vor allem aber ist nachteilig, daß die angestrebte Schutzwirkung der Galactose durch Molkeeiweiß behindert werden kann. Dem trägt eine Weiterbildung der Erfindung Rechnung, nach der beim Einsatz von Molke als Ausgangsprodukt zur Gewinnung des zum Zahnschutz bestimmten LHP, das in dem Ausgangsprodukt enthaltene Protein entfernt wird.

Das kann geschehen, indem man von vornherein von entsprechend proteinfreier Molke ausgeht oder in der Molke vor der Hydrolysation oder nach der Hydrolysation die Proteine entfernt, vorzugweise durch Ausfällen und Abfiltern.

Absolute Proteinfreiheit ist in diesem Zusammenhang nicht erforderlich und auch nicht gemeint und mit tragbarem Aufwand auch nicht möglich. Gemeint ist relative Proteinfreiheit, so wie sie großtechnisch wirtschaftlich erzielbar ist, etwa unterhalb eines Restproteingehaltes von 0,5 %.

Man kann bei der Herstellung des LHP von flüssiger Molke ausgehen, so wie sie bei Milchverarbeitung, zum Beispiel zu Käse, anfällt. Praktisch und deshalb bevorzugt ist es aber in aller Regel, von Molkepulver, das heißt, getrockneter flüssiger Molke auszugehen, die dann wieder aufgelöst wird, weil Molkepulver verhältnismäßig preiswert gehandelt wird und lagerfähig ist.

Verfahren zur Verbesserung von Nahrungsmitteln beziehungsweise Getränken der eingangs genannten Art sind in den Unteransprüchen beschrieben.

Die Erfindung wird nun anhand einiger Beispiele näher erläutert.

## BEISPIEL 1

Herstellung von LHP aus Lactose

### Eingesetzte Lactose

10 kg (Kilogramm) Lactosepulver wird in 30 l (Liter) 18°C warmem Wasser gelöst. Der gewonnenen Lösung werden 70 l 70°C warmes Wasser zugesetzt sowie 100 g (Gramm) Ascorbinsäure dann wird 20 Minuten gerührt.

### pH-Wert-Einstellen

Die Lösung wird auf 20°C abgekühlt und mit Milchsäure auf pH 4,5 eingestellt.

### Hydrolysieren

Die Lösung wird bei einer Temperatur von 32°C mit 3 g des Enzyms EC 3.2.1.23 versetzt und 40 Stunden unter Rühren auf 32°C gehalten. Dann sind ca. 80 % TM der Lactase hydrolisiert.

### LHP Einsatzfertig

Die hydrolisierte Lösung wird durch Gefriertrocknen auf 99 % TM eingetrocknet. Das so gewonnene LHP-Pulver enthält an Trockensubstanz:

| Lactose | 25 % |
|---|---|
| Galactose | 39 % |
| Glucose | 36 %. |

3

BEISPIEL 2

Herstellung von LHP aus Molkepulver

Molkepulver wird gewonnen durch Trocknen von bei der Käseherstellung gewonnener Molke. Das Molkepulver hat folgende Trockenbestandteile: Protein 3,3 %; Fett 1,4 %; Lactose 84,3 %; Mineralstoffe 8,2 %. 10 kg Molkepulver wird in 30 l 18°C warmem Wasser gelöst. Der gewonnenen Lösung werden 70 l 70°C warmes Wasser zugesetzt, sowie 100 g Ascorbinsäure.

Proteinabscheiden

Die Molke wird 30 Minuten bei 70°C gerührt, das dabei aus fallende Protein wird abfiltriert. Das Filtrat wird dann nochmals 30 Minuten bei 70°C gerührt und dann auf 20°C abgekühlt.

pH-Wert-Einstellen

Das abgekühlte Filtrat wird mit Milchsäure auf pH 4,5 eingestellt.

Hydrolysieren

Das Filtrat wird bei einer Temperatur von 32°C mit 3 g des Enzyms EC 3.2.1.23 versetzt. Die Lösung wird 40 Stunden unter Rühren auf 32°C gehalten. DAnn sind ca. 80 % TM der Lactase hydrolisiert.

LHP Einsatzfertig

Die hydrolisierte Lösung wird durch Gefriertrocknen auf 99 % TM eingetrocknet. Das so gewonnene LHP-Pulver enthält an Trockensubstanz:

| Protein | 0,5 % |
|---|---|
| Fett | 1,5 % |
| Lactose | 21,8 % |
| Galactose | 35,0 % |
| Glucose | 33,0 % |
| Mineralstoffe | 8,2 %. |

BEISPIEL 3

Herstellung von LHP aus Molkepulver

Eingesetzte Molke, Proteinabscheiden, pH-Wert-Einstellen, Hydrolysieren

wie Beispiel 2,

LHP-Einsatzfertig

Die hydrolisierte Lösung wird zu einem LHP-Konzentrat eingeengt. Das so gewonnene LHP-Konzentrat enthält an

| Protein | 0,5 Gewichtsteile, |
|---|---|
| Fett | 1,5 Gewichtsteile, |
| Lactose | 21,8 Gewichtsteile, |
| Galactose | 35,0 Gewichtsteile, |
| Glucose | 33,0 Gewichtsteile, |
| Mineralstoffe | 8,2 Gewichtsteile und |
| Wasser | 85,0 Gewichtsteile. |

BEISPIEL 4

Herstellung von LHP aus Molkepulver

Eingesetzte Molke, Proteinabscheiden, pH-Wert-Einstellen

wie Beispiel 2.

Hydrolysieren

Das Filtrat wird bei einer Temperatur von 32° C mit 300 g des unter der Warenbezeichnung Plexazym LA1 bekannten trägergebundenen Enzympräparates versetzt. Die Lösung wird 40 Stunden unter Rühren auf 32° C gehalten. Dann sind ca. 80 % TM der Lactase hydrolisiert. Das Enzympräparat wird nun durch Filtration abgetrennt.

LHP Zusammensetzung und LHP Einsatzfertig

wie Beispiel 2.

BEISPIEL 5

Herstellung von LHP aus Molke

Eingesetzte Molke

Bei der Käseherstellung gewonnene Molke mit einem Gesamttrockengewichtsgehalt von 6,4 % TM. Davon entfallen auf den Proteingehalt 0,3 % TM, den Fettgehalt 0,1 % TM, den Lactosegehalt 5,4 % TM und den Mineralstoffehalt 0,6 % TM.

Proteinabscheiden, pH-Wert-Einstellen, Hydrolysieren und LHP Einsatzfertig

wie Beispiel 2.

LHP Zusammensetzung

Das so gewonnene LHP enthält weniger als 0,1 % Protein, 0,1 % Fett, 1,2 % Lactose, 2,1 % Galactose, 1,9 % Glucose und 0,6 % Mineralstoffe. Der Rest ist Wasser.

## BEISPIEL 6

Herstellung von LHP aus wiederaufgelöstem Molkepulver

Eingesetzte Molke, Proteinabscheiden, pH-Wert-Einstellen, Hydrolysieren und LHP Zusammensetzung

wie Beispiel 2.

## LHP Einsatzfertig

Das gewonnene LHP wird dann in einem Dünnfilmverdampfer eingeengt auf 50 % TM, einer Entkeimungsfiltration unterzogen. Der so gewonnene LHP-Sirup wird durch Gefriertrocknung getrocknet auf einen Feuchtegehalt von weniger als 0,3 % und fein pulverisiert.

## BEISPIEL 7

Nahrungsmittel mit LHP

Zur Herstellung einer süßen Backware wird ein Teig nach einer bekannten Rezeptur angerührt und mit Kristallzucker gesüßt. Zusätzlich zu der bekannten Rezeptur wird LHP-Pulver nach Beispiel 1 in den Teig eingemischt, und zwar auf 100 TGT Saccharose 18 TGT LHP-Pulver.

## BEISPIEL 8

Getränk mit LHP

1 l Vollmilch wird mit 100 g Saft von ausgepreßten Orangen vermischt. Die Mischung enthält 4 g TM Saccharose, 2 g TM Glucose und 3 g Fructose. Zum Süßen werden zugegeben 40 g Kristallzucker und 9 g LHP-Pulver nach Beispiel 1. Die Dosierung des MHP-Pulvers berücksichtigt nicht nur die Menge an zugesetzter Saccharose, sondern auch die bereits vorhandene Saccharose, Glucose und Fructose sowie die mit dem MHP-Pulver zugesetzte Glucose.

## BEISPIEL 9

Getränk mit LHP

1 l Orangensaft enthält 40 g Saccharose, 24 g Glucose, 26 g Fructose. Dem werden 13 g LHP-Pulver nach Beispiel 1, 8 g Trikaliumcitrat und 2 g Dikaliumhydrogenphosphat zugesetzt. Es entsteht auf diese Weise ein Puffersystem mit dem Pufferbereich von pH 6 bis pH 7,5.

## BEISPIEL 10

Getränk mit LHP

Es werden eingesetzt:

| Orangensaftkonzentrat die 65 kg Zucker enthalten, | 102,70 kg, |
| LHP-Konzentrat gewonnen nach Beispiel 3 | 31,00 kg |
| Di-Natriumhydrogenphosphat | 0,46 kg |
| Kaliumdihydrogenphosphat | 7,93 kg |
| Orangenaroma | 0,06 kg |
| kristalline Saccharose | 40,00 kg. |

Die Einsatzmengen werden auf 1000 l vollentsalztes Wasser aufgefüllt und darin gelöst. Der so entstandene Orangensaft enthält 5 TGT Galactose auf 100 TGT übrigen Zucker und hat einen pH 3,6 und einen KaP 6,0 = 41.

BEISPIEL 11

Getränk mit LHP

wie Beispiel 10 mit dem einzigen Unterschied, daß als Puffersubstanz eingesetzt sind:

| Di-Natriumhydrogenphosphat | 0,26 kg |
| Kaliumdihydrogenphosphat | 3,96 kg |

Der so entstandene Orangensaft hat einen KaP 6,0 = 28.

BEISPIEL 12

Getränk mit LHP

wie Beispiel 10 mit dem einzigen Unterschied, daß als Puffersubstanz eingesetzt sind:

| Di-Natriumhydrogenphosphat | 0,15 kg |
| Kaliumdihydrogenphosphat | 2,60 kg |

Der so entstande Orangensaft hat einen KaP 6,0 = 20.

BEISPIEL 13

Getränk mit LHP

wie Beispiel 10 mit dem einzigen Unterschied, daß als Puffersubstanz eingesetzt sind:

| Trikaliumcitrat | 8,00 kg |

Der so entstandene Orangensaft hat einen KaP 6,0 = 10.

BEISPIEL 14

Getränk mit LHP

wie Beispiel 10 mit dem einzigen Unterschied, daß als Puffersubstanz eingesetzt sind:

| Trikaliumcitrat | 4,00 kg |
|---|---|
| Kaliumdihydrogenphosphat | 4,00 kg |

Der so entstandene Orangensaft hat einen KaP 6,0 = 25.


BEISPIEL 15


Getränk mit LHP


Es werden eingesetzt:

| Mehrfrucht-Getränke-Grundstoff die 65 kg Zucker enthalten, | 125,00 kg, |
|---|---|
| LHP-Konzentrat gewonnen nach Beispiel 3 | 31,00 kg |
| Di-Natriumhydrogenphosphat | 0,46 kg |
| Kaliumdihydrogenphosphat | 8,10 kg |
| kristalline Saccharose | 40,00 kg. |

Die Einsatzmengen werden auf 1000 l vollentsalztes Wasser aufgefüllt und darin gelöst. Der so entstandene Mehrfruchtsaft enthält 5 TGT Galactose auf 100 TGT übrigen Zucker und hat einen pH 3,9 und einen KaP 6,0 = 37.


BEISPIEL 16

Die nach Beispiel 1, 2, 3, 4, 5 oder 6 gewonnene LHP wird bei der Herstellung von zuckerhaltigen Nahrungsmitteln zusammen mit dem Zucker zugesetzt. Der Zusatz erfolgt in Abhängigkeit vom Zuckergehalt des fertigen Nahrungsmittels und vom Galactosegehalt des LHP, und zwar derart, daß auf 100 Trockengewichtsteile (TGT) Gehalt an Saccharose, Glucose, Fructose und Maltose, bezogen auf das fertiggestellte Nahrungsmittel, 2 bis 10 TGT, vorzugsweise 4,5 bis 5,5 TGT, Galactose im zugesetzten LHP eingesetzt sind, und zwar unter Anrechnung der eventuell im Nahrungsmittel bereits enthaltenen Galactose und sonstigen Zucker.

Die Beispiele 7 bis 15 sind abänderungsfähig dadurch, daß eine größere oder kleinere Menge an Galactose beziehungsweise LHP zugesetzt wird im Rahmen der im Anspruch 1 und Beispiel 16 für den Galactosegehalt angegebenen Grenzen.

Dementsprechend kann bei den Beispielen 10 bis 15 die zugesetzte Molkekonzentratmenge abgeändert werden beispielsweise auf 15,00 kg, 20,00 kg, 40,00 kg oder 60,00 kg.

Weitere Beispiele unterscheiden sich von den vorangegangenen Beispielen durch die aus den nachfolgenden Tabellen ersichtlichen Angaben.

8

TABELLE 1 - Teil 1

| BEISPIEL | | 17 | 18 | 19 | 20 |
|---|---|---|---|---|---|
| **Ausgangsprodukt** | | | | | |
| A | Molke | nein | nein | nein | nein |
| B | Lactose | ja | ja | ja | ja |
| C | Trockengehalt in %TG | 98 | 98 | 98 | 98 |
| D | davon Protein in % | 0 | 0 | 0 | 0 |
| E | Fett in % | 0 | 0 | 0 | 0 |
| F | Lactose in % | 100 | 100 | 100 | 100 |
| G | Mineralstoffe in % | 0 | 0 | 0 | 0 |
| H | Ascorbinsäure in % | 1,0 | 2,0 | 1,5 | 3,0 |
| **Proteinabscheiden** | | | | | |
| I | Behandlungstemperatur °C | --- | --- | --- | --- |
| J | Behandlungsdauer Minuten | --- | --- | --- | --- |
| **pH-Wert-Einstellen** | | | | | |
| K | pH | 4,5 | 5,5 | 6,5 | 7,0 |
| **Hydrolysieren** | | | | | |
| L | eingesetztes Enzym:EC 3.1.2.23 | immo-bili-siert | immo-bili-siert | frei | frei |
| M | Menge eingesetztes Enzym in g/l | 3,o | 10 | 0,1 | 1,0 |
| N | Behandlungstemperatur °C | 15 | 30 | 40 | 55 |
| O | Behandlungsdauer Stunden | 60 | 40 | 8 | 20 |
| **LHP Zuckergehalt** | | | | | |
| P | Lactose in %TM | 0,6 | 1,6 | 4,0 | 12,8 |
| Q | Glucose in %TM | 1,7 | 3,2 | 6,0 | 9,6 |
| R | Galactose in %TM | 1,7 | 3,2 | 6,0 | 9,6 |
| **Bemerkung** | | | | | |
| S | Im übrigen nach Beispiel | 1 | 1 | 1 | 1 |

TABELLE 1 - Teil 2

| BEISPIEL | 21 | 22 | 23 | 24 |
|---|---|---|---|---|
| **Ausgangsprodukt** | | | | |
| A Molke | ja | ja | ja | ja |
| B Lactose | nein | nein | nein | nein |
| C Trockengehalt in %TG | 5,9 | 11,8 | 25,0 | 50,0 |
| D davon Protein in % | 0,3 | 0,6 | 2,0 | 4,0 |
| E Fett in % | 0,1 | 0,2 | 0,5 | 1,0 |
| F Lactose in % | 4,0 | 8,0 | 16,0 | 32,0 |
| G Mineralstoffe in % | 0,5 | 1,0 | 2,5 | 5,0 |
| H Ascorbinsäure in % | 1,0 | 2,0 | 1,5 | 3,0 |
| **Proteinabscheiden** | | | | |
| I Behandlungstemperatur °C | 70 | 80 | 90 | 100 |
| J Behandlungsdauer Minuten | 30 | 25 | 15 | 10 |
| **pH-Wert-Einstellen** | | | | |
| K pH | 4,5 | 5,5 | 6,5 | 7,0 |
| **Hydrolysieren** | | | | |
| L eingesetztes Enzym:EC 3.1.2.23 | immo-bili-siert | immo-bili-siert | frei - | frei - |
| M Menge eingesetztes Enzym in g/l | 3,o | 10 | 0,1 | 1,0 |
| N Behandlungstemperatur °C | 15 | 30 | 40 | 55 |
| O Behandlungsdauer Stunden | 60 | 40 | 8 | 20 |
| **LHP Zuckergehalt** | | | | |
| P Lactose in %TM | 0,6 | 1,6 | 4,0 | 12,8 |
| Q Glucose in %TM | 1,7 | 3,2 | 6,0 | 9,6 |
| R Galactose in %TM | 1,7 | 3,2 | 6,0 | 9,6 |
| **Bemerkung** | | | | |
| S Im übrigen nach Beispiel | 4 | 6 | 5 | 6 |

**Ansprüche**

1. Nahrungsmittel oder Genußmittel, vorzugsweise Getränk, für menschliche Ernährung

mit einem Gehalt an Zucker,
mit einem Gehalt an Galactose in Höhe eines Bruchteils des übrigen Zuckergehaltes zur Vermeidung der zahnschädigenden Wirkungen dieses Zuckers, dadurch gekennzeichnet,
daß auf 100 Trockengewichtsteile (TGT) Gehalt an übrigem Zucker (Gesamtzucker, ausgenommen Galactose) 2 bis 10 TGT, vorzugsweise 5 TGT, Galactose als Zusatz eingesetzt werden, und zwar unter Anrechnung der eventuell bereits vorhandenen Galactose.

2. Nahrungsmittel oder Genußmittel vorzugsweise Getränk nach Anspruch 1, gekennzeichnet,
durch einen sauren pH, vorwiegend hervorgerufen durch organische Säuren, und dadurch
daß als Puffersubstanz Phosphat und/oder Citrat, vorzugsweise in Form von Alkalisalz, eingesetzt ist.

3. Nahrungsmittel oder Genußmittel vorzugsweise Getränk nach Anspruch 2, dadurch gekennzeichnet,
daß die Pufferkapazität KaP definiert ist nach der Gleichung KaP x = y, wobei KaP x die Pufferkapazität KaP für pH x ist und wobei y die Anzahl der ml (Milliliter) an 0,1 nHCL (Normal-Salzsäure) angibt, die nötig sind, um 100 ml einer auf pH 7 eingestellten Lösung auf pH x umzustellen, und
daß nach dieser Definition die Pufferkapazität KaP 6,0 eingestellt ist auf 10 bis 45, vorzugsweise 20 bis 41.

4. Nahrungsmittel oder Genußmittel vorzugsweise Getränk nach einem der vorhergehenden Ansprüche, dadurch gekennzeichnet,
daß die Galactose in Form eines Lactosehydrolysationsproduktes (LHP) eingesetzt ist, das gewonnen ist, indem Lactose mit Beta-Galactosidase hydrolysiert ist und die so behandelte Lactose mit ihrem gesamten Zuckergehalt als LHP eingesetzt ist.

5. Nahrungsmittel oder Genußmittel vorzugsweise Getränk nach Anspruch 4, dadurch gekennzeichnet,
daß die Hydrolysation unter Einsatz von Mikroorganismen erfolgt und
daß die Mikroorganismen nach der Hydrolysation und vor dem Einsatz des LHP entfernt sind.

6. Nahrungsmittel oder Genußmittel vorzugsweise Getränk nach Anspruch 4, dadurch gekennzeichnet,
daß in dem eingesetzten LHP 60 bis 85%, vorzugsweise 75 bis 80%, der Lactose hydrolysiert ist.

7. Nahrungsmittel oder Genußmittel vorzugsweise Getränk nach Anspruch 4, dadurch gekennzeichnet,
daß ein LHP eingesetzt ist, das durch Hydrolysation von Molke - flüssige Molke oder wiederaufgelöstes Molkepulver -gewonnen ist und das kein Protein enthält.

8. Verfahren zur Verbesserung eines Nahrungsmittels oder Genußmittels vorzugsweise Getränks mit einem Gehalt an Saccharose und einem als Zahnschutz dienenden Zusatz von Galactose in Form eines durch enzymatische Spaltung gewonnenen LHP nach einem der vorhergehenden Ansprüche, dadurch gekennzeichnet,
daß Lactose mit Beta-Galactosidase behandelt wird, bis 60 bis 85%, vorzugsweise 75 bis 80%, der Lactose hydrolysiert sind, und
daß das so gewonnene LHP dem Nahrungsmittel beziehungsweise Getränk zugesetzt wird in einer Menge, so daß 2 bis 10 TGT, vorzugsweise 4,5 bis 5,5 TGT, Galactose auf 100 TGT übriger Zuckergehalt kommt.

9. Verfahren nach Anspruch 8, dadurch gekennzeichnet,
daß 10 kg Lactosepulver, gelöst in 50 bis 150 l Wasser, mit einem Zusatz von 50 bis 200 g (Gramm), vorzugsweise 100 g, Ascorbinsäure auf 60 bis 80° C, vorzugsweise 70° C, erwärmt und 15 bis 60 Minuten gerührt werden,
daß dann der pH-Wert eingestellt wird auf pH 3 bis 9, vorzugsweise pH 6,3 bis 6,7, die Temperatur abgesenkt wird auf 15 bis 55° C, vorzugsweise 32° C, Beta-Galactosidase zugesetzt wird und die Lösung unter Rühren bei dieser Temperatur gehalten wird, bis 60 bis 85%, vorzugsweise 75 bis 80%, der Lactose hydrolysiert ist und
daß das so gewonnene LHP bei der Herstellung des Nahrungsmittels diesem vorzugsweise in der gleichen Herstellungsstufe mit den gleichen Maßnahmen zugesetzt wird wie die Saccharose oder andere Zucker.

10. Verfahren nach Anspruch 8, dadurch gekennzeichnet, daß Molke eingesetzt wird in natürlicher flüssiger Form oder als wiederaufgelöstes Molkepulver, wofür 10 kg Molkepulver mit einem Lactosegehalt von 80 bis 88% TGT, gelöst in 50 bis 150 l (Liter) Wasser, mit einem Zusatz von 50 bis 200 g, vorzugsweise 100 g, Ascorbinsäure auf 60 bis 80° C, vorzugsweise 70° C, erwärmt und 15 bis 60 Minuten gerührt werden,
daß dann der pH-Wert der eingesetzten Molke eingestellt wird auf pH 3 bis 9, vorzugsweise pH 6,3 bis 6,7, die Temperatur abgesenkt wird auf 15 bis 55° C, vorzugsweise 32° C, Beta-Galactosidase zugesetzt wird und die Lösung unter Rühren bei dieser Temperatur gehalten wird, bis 60 bis 85%, vorzugsweise 75 bis 80%, der Lactose hydrolysiert ist und ein LHP gewonnen ist,
daß - um ein proteinfreies LHP zu gewinnen - entweder von proteinfreiem Molkepulver ausgegangen wird und/oder aus dem in Wasser aufgelöstem Molkpulver vor der Hydrolysation und/oder nach der Hydrolysation die Proteine entfernt werden, vorzugsweise durch Ausfällen und Abfiltrieren, und
daß das so gewonnene, proteinfreie LHP bei der Herstellung des Nahrungsmittels diesem vorzugsweise in

EP 0 349 712 A1

der gleichen Herstellungsstufe mit den gleichen Maßnahmen zugesetzt wird wie die Saccharose oder andere Zucker.

12

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CA-A-1 225 864 (IMPERIAL BIOTECHNIQUES) * Ansprüche 1-34 * --- | 1,4-8 | A 23 L 1/30<br>A 23 C 9/12<br>C 13 K 13/00<br>A 23 L 2/26 |
| D,X | EP-A-0 184 121 (BIODYN) * Ansprüche 1-15; Seite 15, Zeilen 24-30 * ----- | 1-3,8 | |

RECHERCHIERTE
SACHGEBIETE (Int. Cl.4)

A 23 L
A 23 C
C 13 K

Der vorliegende Recherchenbericht wurde für alle Patentansprüche erstellt

| Recherchenort | Abschlußdatum der Recherche | Prüfer |
|---|---|---|
| DEN HAAG | 07-07-1989 | VAN MOER A.M.J. |

EPO FORM 1503 03.82 (P0403)